# EUROPEAN PATENT APPLICATION

(11) **EP 1 884 235 A1**
(43) Date of publication of application: **06.02.2008**
(21) Application number: 06732369.1
(22) Date of filing: 26.04.2006
(51) Int. Cl.: A61K 31/198, A61K 38/22, A61P 7/00, A61P 7/06, A61P 13/12, A61P 43/00

(54) **MYELOERYTHROID PROGENITOR DIFFERENTIATION INDUCER**

(30) Priority: 26.04.2005 JP 2005128204
(71) Applicant: Ajinomoto Co., Inc., Tokyo 104-8315 (JP)
(72) Inventor: TAKESHITA, Sen c/o AJINOMOTO CO., INC., Kawasaki-shi, Kanagawa 2108681 (JP); ETO, Yuzuru c/o AJINOMOTO CO., INC., Kawasaki-shi, Kanagawa 2108681 (JP); TAKAHARA, Yoshiyuki c/o AJINOMOTO CO., INC., Kawasaki-shi, Kanagawa 2108681 (JP); KAMIYA, Kiyonobu c/o AJINOMOTO CO., INC., Kawasaki-shi, Kanagawa 2108681 (JP); TASHIRO, Kazumi c/o AJINOMOTO CO., INC., Kawasaki-shi, Kanagawa 2108681 (JP); SUGIYAMA, Maki c/o AJINOMOTO CO., INC., Kawasaki-shi, Kanagawa 2108681 (JP); YAMAMOTO, Hiroshi c/o AJINOMOTO CO., INC., Kawasaki-shi, Kanagawa 2108681 (JP)
(74) Representative: Nicholls, Kathryn Margaret
(86) International application number: PCT/JP2006/308763
(87) International publication number: WO 2006/115274

(57) **Abstract**

There are provided a bone marrow erythroid progenitor cell(s) differentiation inducer comprising arginine as an active ingredient and a therapeutic agent for anemia comprising the differentiation inducer. This differentiation inducer is excellent in that it has high safety and versatility, and can be orally administered.

## Description

### Field of the Invention

The present invention relates to a bone marrow erythoid progenitor cell(s) differentiation inducer, a therapeutic agent for anemia using the same. The present invention also relates to a hematopoietic factor production promoter and a method of screening an enhancer or inhibitor for the hematopoietic factor production promoter.

### Background of Invention

Erythropoiesis is an essential process required to maintain homeostasis of the number of red blood cells. Red blood cells have an average life span of about 120 days in human and worn out red blood cells are continuously removed from the circulation system. Therefore, about 100 billion red blood cells are newly produced daily in adults. There have been many investigations of generation of red blood cells, which are described in a number of books. As a typical example, a summary is excerpted from "Hematology, Chugai Igakusha", which will be shown below.

There are multipotent stem cells capable of differentiating into many different types of blood cell systems in the bone marrow, and a part of the multipotent stem cells differentiate into erythroid progenitor cells determined to differentiate into the erythroid system. The most primitive identifiable erythroid progenitor cells are the burst forming unit-erythroid (BFU-E) and the colony forming unit-erythroid (CFU-E), which is a more differentiated cell. Beyond the CFU-E stage, the cells differentiate into proerythroblasts, basophilic erythroblasts, polychromatic erythroblasts, and orthochromatic erythroblasts while dividing, and reticulocytes differentiate into mature red blood cells by enucleation. At the BFU-E and CFU-E stages, the direction of differentiation into the erythroid system is completely determined, therefore the cells never differentiate into a blood cell system other than the erythroid system. Thus, the number of BFU-E and CFU-E increases, erythropoiesis is promoted.

As for erythropoiesis, homeostasis is principally regulated by erythropoietin (EPO), which is a hematopoietic factor. EPO is mainly produced in the kidney and circulates in the blood and acts on CFU-E in the bone marrow and stimulates proliferation and differentiation of CFU-E, thereby promoting erythropoiesis. When EPO is not produced at a normal level and running short, CFU-E decreases and erythropoiesis is reduced to cause anemia. Anemia is the pathological consequence of insufficient hemoglobin levels to meet the oxygen transport requirements of the body and causes clinical symptoms such as less incentive to work, fatigability, shortness of breath, lightheadedness and palpitation, therefore there is a demand for improving such symptoms. It is known that various diseases cause anemia due to insufficient EPO, and the most classical example is a kidney disease. In patients with chronic renal failure, EPO production is reduced due to renal damage, whereby the patients exhibit anemia. Many of the patients with chronic renal failure are dialysis patients who require frequent dialysis for renal function replacement, and 90% of the dialysis patients have anemia. At present, as a method of treating anemia, administration of recombinant human EPO (rHuEPO) is widely used, and 90% of the dialysis patients are administered with rHuEPO. In many of the patients, an effect on improving anemia has been confirmed.

The effect of rHuEPO on improving anemia is high, however, several problems have arisen accompanying the expansion of the clinical use thereof. One is the cost for long-term treatment thereof. Typical dose of rHuEPO is 9000 IU/week at most, which costs about 12,000 yen on an NHI drug price basis. However, unless the primary disease-causing anemia is treated, it is administered over a long period of time. Therefore, the burden of patients and health care system are great in terms of the cost. Further, because EPO has to be intravenously administered, the patients need to go to the hospital every time they receive the administration, etc., which is also a cause why the burden of patients is great. Further, it is known that there are about 10 to 20% of patients whose EPO level in the blood is increased by the administration of rHuEPO, but whose reactivity against EPO is low, therefore who need a high dose of rHuEPO for improving anemia or whose anemia is hardly improved at all (these patients are called EPO-unresponsive patients). It is considered that for improving anemia of the EPO-unresponsive patients, a bone marrow erythoid progenitor cell(s) differentiation inducer, which increases CFU-E based on the mechanism of action different from that of EPO, is effective. Further, by the concomitant use of the bone marrow erythoid progenitor cell(s) differentiation inducer, an effect on reducing the required dose of rHuEPO is also expected.

Further, for an individual who collects and stores his/her own blood for the future use (an individual who stores his/her own blood), the bone marrow erythoid progenitor cell(s) differentiation inducer can be used as a means of promoting hematopoietic action promptly after the collection of blood.

On the other hand, it is known that proliferation and differentiation of erythroid progenitor cells are induced by a plurality of hematopoietic factors other than EPO. It is known that activin and IGF-1, each of which is a hematopoietic factor, act on CFU-E synergistically with EPO and induce proliferation and differentiation thereof (Blood, 1992, Nov 15; 80(10): 2503-12). It is also known that SCF, which is a hematopoietic factor, acts on BFU-E, which is an erythroid progenitor cell less differentiated than CFU-E and induces proliferation and differentiation thereof (Blood, 1991, Oct. 15; 78(8): 1975-80).

These hematopoietic factors, activin, IGF-1 and SCF act on a receptor different from that of EPO and increase CFU-E, therefore it is expected that anemia is improved by administration thereof to EPO-unresponsive patients. However, because administration of a recombinant protein of a hematopoietic factor is parenteral administration like EPO, the burden of patients is great, for example, the patients need to go to the hospital every time they receive the administration, etc. Further, it is known that when a plurality of hematopoietic factors act on concomitantly, a synergistic action thereof are exhibited in general, however, the concomitant use of a plurality of hematopoietic factors makes the burden of patients great in terms of cost.

Accordingly, it is expected that if a medicament that has an action of promoting the production of activin, IGF-1 and SCF simultaneously is discovered, the medicament will become a therapeutic agent for anemia that solves the current clinical problems.

In non-patent document 1, it is described that the number of hemoglobins and red blood cells was increased by repeatedly administering arginine to normal rats. However, it is not known whether it is caused by an effect on enhancing erythroid regeneration (erythropoiesis), or an effect on prolonging the life span of red blood cells, that is, the mechanism of action is not known. Therefore, a clinical effectiveness, for example, the type of anemia in which the inducer is effective, is not clear. In non-patent document 2, it is described that the condition of anemia was improved (the number of red blood cells came close to a normal level) by administering arginine to patients with renal anemia, and the mechanism of action thereof resides in the promotion of renal EPO production by arginine. Accordingly, it can be inferred naturally and easily that the phenomenon of increasing the number of red blood cells observed in normal rats described in the non-patent document 1 is caused by the promotion of renal EPO production in the rats by arginine. That is, for researchers in this field, it is difficult to predict that arginine has a hematopoietic action by directly acting on the bone marrow to induce proliferation of erythroid progenitor cells. Further, it is a natural consequence that the researchers think that the type of anemia in which arginine is effective is limited to one in which the promotion of EPO production is involved in the therapeutic effect. In this way, the researchers never thought that arginine has a myelopoietic action, arginine can also be applied to a treatment of anemia for EPO-unresponsive patients, the dose of EPO can be reduced by a synergistic effect with EPO and the like.

In non-patent document 3, it is reported that by adding arginine, proline, threonine and tryptophane to a medium for porcine primary hepatocyte culture at a high concentration, gene expression of IGF-1 increases. This study was performed by limiting the actions of amino acids on hepatocytes, and there is no description on an action on cells other than hepatocytes or an action on erythropoiesis.

In non-patent document 4, it is reported that when arginine is added to MC3T3-E1 cell, which is an osteoblast-like cell line, the secretion amount of IGF-1 increases, and when arginine is added at a higher concentration, the gene expression increases.

It is described that because osteoblasts are involved in the formation of bone, there may be a possibility that arginine promotes the formation of bone by enhancing IGF-1 production. That is, this study was performed by paying attention to the action on bone metabolism regulation, and there is no description on an action on erythropoiesis in the document.
Non-patent document 1 : Int. J. Toxicol., 2004, 23: 101-105
Non-patent document 2 : Igakuno Ayumi 2004, 211, No. 8
Non-patent document 3 : J. Nutr., 1999, 129; 1298-1306
Non-patent document 4 : Bone, 1998, 23; 103-109

### Disclosure of the Invention

An object of the present invention is to provide a bone marrow erythoid progenitor cell(s) differentiation inducer.

Another object of the present invention is to provide a therapeutic agent for anemia.

Another object of the present invention is to provide a hematopoietic factor production promoter.

Another object of the present invention is to provide a method of screening an enhancer or inhibitor for the hematopoietic factor production promoter.

The present inventors have made a search and investigation of a medicament that can solve the above-mentioned problems, and as a result, they newly discovered that arginine, which is an amino acid, has an action of increasing CFU-E, thus the present invention has been completed.

The present inventors also newly discovered that arginine, which is an amino acid, has an action of increasing CFU-E via the promotion of production of activin, SCF and IGF-1, thus the present invention has been completed.

More specifically, in a method of measuring CFU-E, the in vitro colony assay method using a methylcellulose semi-solid medium is generally used, and it is the most suitable experimental system for discovering an action of increasing CFU-E. The present inventors employed this method and measured the number of CFU-E colonies when 600 µM arginine was added to isolated mouse bone marrow cells, and as a result, they found that the number of CFU-E significantly increases compared with the case of an average concentration of arginine in the blood, 170 µM. In the case of oral administration to rats, the blood arginine concentration reaches about 600 µM by a single- dose administration of arginine at 1.2 g/kg. Therefore, it is expected that the number of CFU-E increases in a dose- dependent manner by the oral administration. Also in human, it is expected that the number of CFU-E increases as the concentration of arginine in the blood increases by the oral administration.

Accordingly, the following aspects of the invention are provided.
(1) A bone marrow erythoid progenitor cell(s) differentiation inducer, comprising arginine as an active ingredient.
(2) A therapeutic agent for anemia, comprising the differentiation inducer according to (1).
(3) The therapeutic agent for anemia according to (2), which is for hemodialysis or peritoneal dialysis.
(4) The therapeutic agent for anemia according to (2), which is for oral administration.
(5) The therapeutic agent for anemia according to (2), which is for enteral administration.
(6) The therapeutic agent for anemia according to any one of (2) to (5), which is for treating renal anemia.
(7) The therapeutic agent for anemia according to any one of (2) to (5), which is for erythropoietin- unresponsive patients.
(8) The therapeutic agent for anemia according to any one of (2) to (7), further comprising erythropoietin in combination.
(9) The therapeutic agent for anemia according to (8), wherein the erythropoietin-like substance(s) is erythropoietin.
(10) The therapeutic agent for anemia according to (8), wherein the erythropoietin-like substance(s) is an erythropoietin mimic peptide.
(11) The therapeutic agent for anemia according to (8), wherein the erythropoietin-like substance(s) is an erythropoietin production promoter.
(12) The therapeutic agent for anemia according to any one of (8) to (11), which is a combination preparation.
(13) The therapeutic agent for anemia according to (8), which is a kit composed of a medicament comprising arginine and a medicament comprising erythropoietin-like substance(s).
(14) The therapeutic agent for anemia according to (13), wherein the erythropoietin-like substance(s) is erythropoietin.
(15) The therapeutic agent for anemia according to (13), wherein the erythropoietin-like substance(s) is an erythropoietin mimic peptide.
(16) The therapeutic agent for anemia according to (13), wherein the erythropoietin-like substance(s) is an erythropoietin production promoter.
(17) An agent for enhancing the effect of erythropoietin-like substance(s), comprising arginine as an active ingredient.
(18) The agent according to (17), wherein the erythropoietin-like substance(s) is erythropoietin.
(19) The agent according to (17), wherein the erythropoietin-like substance(s) is an erythropoietin mimic peptide.
(20) The agent according to (17), wherein the erythropoietin-like substance(s) is an erythropoietin production promoter.
(21) An agent for reducing the dose of erythropoietin-like substance(s), comprising arginine as an active ingredient.
(22) The agent according to (21), wherein the erythropoietin-like substance(s) is erythropoietin.
(23) The agent according to (21), wherein the erythropoietin-like substance(s) is an erythropoietin mimic peptide.
(24) The agent according to (21), wherein the erythropoietin-like substance(s) is an erythropoietin production promoter.
(25) A hematopoiesis promoter for an individual who stores his/her own blood, comprising arginine as an active ingredient.
(26) A hematopoietic factor production promoter comprising arginine as an active ingredient.
(27) The hematopoietic factor production promoter according to (26), wherein the hematopoietic factor is at least one selected from the group consisting of activin, SCF and IGF-1.
(28) The hematopoietic factor production promoter according to (26), which promotes production of hematopoietic factor in the bone marrow.
(29) A method of screening an enhancer for the hematopoietic factor production promoter according to (26), comprising the steps of:
   comparing hematopoietic factor production- promoting activities when arginine or arginine and a test substance is/are added to a hematopoietic factor production-promoting activity measurement system which is for measuring a hematopoietic factor production-promoting activity; and
   selecting the test substance exhibiting a higher hematopoietic factor production-promoting activity when the test substance is added.
(30) A method of screening an inhibitor for the hematopoietic factor production promoter according to (26), comprising the steps of:
   comparing hematopoietic factor production-promoting activities when arginine or arginine and a test substance is/are added to a hematopoietic factor production-promoting activity measurement system which is for measuring a hematopoietic factor production-promoting activity; and
   selecting the test substance exhibiting a lower hematopoietic factor production-promoting activity when the test substance is added.

### Brief Description of the Drawings

Fig. 1 shows the CFU-E-colony stimulating activity of arginine. Figure 1(A) shows the CFU-E increasing action of arginine under the existence of 1U/mL of EPO, which is a maximal action concentration. Figure 1(B) shows that 0.5U/mL or more of EPO is a maximal action amount in the existence of EPO concentration.
Figure 2 shows the CFU-E increasing action of arginine under the existence of an EPO mimic peptide. Figure 2(A) shows the CFU-E increasing action of arginine under the existence of 30 µM of the EPO mimic peptide, which is a maximal action concentration. Figure 2(B) shows that 10 µM or more of the EPO mimic peptide is a maximal action amount in the existence of EPO mimic peptide concentration.
Fig. 3 shows the activin, SCF and IGF-1 production- promoting activity of arginine in bone marrow cells isolated from a renal failure rat.
Fig. 4 shows the inhibition of CFU-E-colony stimulating activity of arginine by inhibitors for hematopoietic factors.

### Description of the Preferred Embodiments

A bone marrow erythoid progenitor cell(s) differentiation inducer and hematopoietic factor production promoters according to the present invention comprises arginine as an active ingredient. The arginine may be arginine and/or a physiologically acceptable salt thereof. Examples of the salt include a hydrochloride, a citrate, a glutamate and the like, however, it is not limited to these. As for an isomer of the active ingredient, any of L-form, D-form and DL-form can be used, however, L-form is preferred because it is naturally occurring. For example, as the arginine, free-form of L-arginine and L-arginine monohydrochloride may be included in combination.

The bone marrow erythoid progenitor cell(s) differentiation inducer and hematopoietic factor production promoters of the present invention can be prepared in any of known forms as well as various forms of pharmaceutical preparations to be discovered in the future for, for example, oral administration, intraperitoneal administration, percutaneous administration, subcutaneous administration, intravenous administration, inhalation and the like. In the myeloerythroid progenitor cell differentiation inducer and hematopoietic factor production promoters of the present invention, arginine can be used alone. However, as a pharmaceutical preparation comprising arginine as an active ingredient, it can be prepared in any of various forms by suitably employing known methods and methods developed in the future.

A method of administering the myeloerythroid progenitor cell differentiation inducer and hematopoietic factor production promoters of the present invention is not particularly limited, however, oral administration is preferred. In this case, the dose varies depending on the hemoglobin level, which becomes an indicator of anemia for a patient to be administered, the age and the like, however, in the case of adults, it is about 0.1 to 12 g, more preferably about 0.5 to 6 g per day.

The bone marrow erythoid progenitor cell(s) differentiation inducer and hematopoietic factor production promoters of the present invention can be used as an active ingredient of a pharmaceutical product to be used for treating or preventing a variety of diseases caused by decrease in erythropoiesis or a constituent of a food or a medical food. Examples of the disease in which the bone marrow erythoid progenitor cell(s) differentiation inducer of the present invention is expected to be effective include renal anemia, iron-deficiency anemia, hemolytic anemia, aplastic anemia, pernicious anemia, bleeding anemia and anemia accompanying a treatment with an anti-cancer agent. Among these diseases, the myeloerythroid progenitor cell differentiation inducer of the present invention is useful for a treatment of renal anemia, particularly EPO-unresponsive renal anemia.

The term "EPO unresponsiveness" as used herein means a state in which a sufficient effect is not expressed even if EPO is administered in an amount at which an effect is expected, and it is not particularly limited to the dose of EPO. It preferably indicates the state wherein the enough effect does not express though 9000 or more IU/week of EPO is administered. The state wherein the enough effect does not express preferably indicates the state wherein the increase of Ht value in 4 to 8 weeks is less than 3%. In general, the definition of an EPO-unresponsive patient varies depending on the race, the lifestyle habit or the like, however, the present invention is not limited to the definition. For example, in the U.S., a patient whose Hb level does not reach 10 to 12 g/dl even if EPO has been administered at 300 to 450 IU/kg/week for 4 to 6 months is defined as an EPO-unresponsive patient in the guideline (Am J Kidney Dis 30 Suppl. 3, 1997). However, in a general dosage regimen, a patient whose Hb level is not increased by 2 g/dl and whose Hb level does not reach 10 to 12 g/dl even if EPO has been administered at 150 to 300 IU/kg/week for 2 months is defined as an EPO- unresponsive patient (the package insert of EPOGEN). In the Europe, a patient whose Ht value does not reach a desired value even if EPO has been subcutaneously administered at 300 IU/kg/week or more is defined as an EPO-unresponsive patient in the guideline (Nephrol Dial Transplant (1999) 14 Suppl. 5: 25-27). In Japan, the required dose of EPO is 9000 IU/week or more, and the case where an increase in the Ht value by 4 to 8-week administration is less than 3% is considered as EPO unresponsiveness. Also in countries other than the above-mentioned countries, the case where an effect on improving anemia cannot be expected even at the recommended maximum dosage regimen is defined as an EPO- unresponsive patient. For such an EPO-unresponsive patient, administration of the bone marrow erythoid progenitor cell(s) differentiation inducer of the present invention is essential.

Among patients administered with EPO other than EPO- unresponsive patients, for patients for whom the required dose of EPO is, preferably 1500 to 9000 IU/week, enhancement of improvement of anemia by the myeloerythroid progenitor cell differentiation inducer of the present invention is expected. Therefore, it is expected that the required dose of EPO at a treatment for improving anemia by EPO alone can be reduced. It is expected that the enhancement of EPO and the reduction of the dose of EPO by administering the myeloerythroid progenitor cell differentiation inducer of the present invention is effective even in the case where sufficient improvement of anemia is not observed for a standard administration period (an increase in the Ht value is less than about 3% for 4 to 8 weeks) at a standard dose of EPO at the initiation of EPO administration. Further, it is expected that the bone marrow erythoid progenitor cell(s) differentiation inducer of the present invention is effective also in the case where sufficient improvement of anemia is not observed for a standard administration period even when the dose of EPO thereafter is increased to the maximum dose to be generally administered. As a method of administration for enhancement of EPO and reduction of the dose of EPO described above, oral administration and parenteral administration can be employed. By administering the bone marrow erythoid progenitor cell(s) differentiation inducer of the present invention, improvement of anemia is expected for patients who undergo or do not undergo dialysis among patients with renal anemia, and as a method of administration, oral administration and parenteral administration can be employed.

An erythropoietin-like substance is the substance which exercises or induces the erythropoietin-like actions in vivo when administering / taking such substance in vivo.

For example, erythropoietin-like substances include erythropoietin, erythropoietin mimic peptides, erythropoietin production promoters or the like, but they are not particularly limited to them.

Erythropoietin may be a natural type, genetical recombinant type, or modified type. For example, in the case of the genetical recombinant type, it may be epoetin alfa (genetical recombination) or epoetin beta (genetical recombination), which are glycoproteins (molecular weight: about 30,000) consisting of 165 amino acid residues (C₈₀₉H₁₃₀₁N₂₂₉O₂₄₀S₅; molecular weight: 18,235.96) produced in Chinese hamster ovary cells by the expression of human erythropoietin cDNA derived from mRNA of human hepatic cells.

An erythropoietin mimic peptide is a peptide which bonds to an EPO receptor and activates it, or works as an EPO agonist. For example, as disclosed in WO96/40749, it includes peptides of 10 to 40 amino acid residues in length, which comprise a sequence of amino acids X3X4GPX6TWX7X8, wherein each amino acid is indicated as the standard one letter abbreviation: X3 is C; X4 is R, H, L or W; X5 is M, F or I; X6 is independently selected from any one of the 20 genetically coded L-amino acids; X7 is D, E, I, L or V; and X8 is C.

More specifically, an EPO mimic peptide includes peptides which comprise the peptides that meet the following requirements as the component(s).
1. A peptide of 10 to 40 amino acid residues in length, which bonds to an EPO receptor and comprises a sequence of amino acids X3X4GPX6TWX7X8 wherein each amino acid is indicated as the standard one letter abbreviation: X6 is independently selected from any one of the 20 genetically coded L-amino acids; X3 is C; X4 is R, H, L or W; X5 is M, F or I; X7 is D, E, I, L or V; and X8 is C.
2. The peptide according to 1, which comprises a sequence of amino acids YX2X3X4X5GPX6TWX7X8 wherein each amino acid is indicated as the standard one letter abbreviation: each X2 and X6 is independently selected from any one of the 20 genetically coded L-amino acids; X3 is C; X4 is R, H, L or W; X5 is M, F or I; X7 is D, E, I, L or V; and X8 is C.
3. The peptide according to 2, which comprises a sequence of amino acids X1YX2X3X4X5GPX6TWX7X8X9X10X11 wherein each amino acid is indicated as the standard one letter abbreviation: each X1, X2, X6, X9, X10 and X11 is independently selected from any one of the 20 genetically coded L-amino acids; X3 is C; X4 is R, H, L or W; X5 is M, F or I; X7 is D, E, I, L or V; and X8 is C.
4. The peptide according to 3, wherein X4 is R or H; X5 is F or M; X6 is I, L, T, M, E or Vi X7 is D or V; X9 is G, K, L, Q, R, S or T; and X10 is A, G, P, R or Y
5. The peptide according to 4, wherein X1 is D, E, L, N, S, T or V; X2 is A, H, K, L, M, S or T; X4 is R or H; X9 is K, R, S or T; and X10 is P.
6. The peptide according to 1, which is selected from the group consisting of the following:
   GGNYMCHFGPITWVCRPGGG;
   GGVYACRMGPITWVCSPLGG;
   VGNYMAHMGPITWVCRPGG;
   GGPHHVYACRMGPLTWIC;
   GGTYSCHFGPLTWVCKPQ;
   GGLYACHMGPMTWVCQPLRG;
   TIAQYICYMGPETWECRPSPKA;
   YSCHFGPLTWVCK;
   YCHFGPLTWVC;
   Ac-GGTYSCHFGPLTWVCKPQGG;
   GGCRIGPITWVCGG;
   LGRKYSCHFGPLTWVCQPAKKD;
   GGTASCHFGPLTWVCKPQGG;
   GGNYYCRFGPITFECHPTGG;
   GGEYLCRMGPMTWVCTPVGG;
   GGLYTCRMGPITWVCLPAGG;
   GGTTSCHFGPLTWVCKPQGG;
   GGTFSCHFGPLTWVCKPQGG;
   GGTYSCHFGALTWVCKPQGG;
   GGTYSCHFGPLAWVCKPQGG;
   GGTYSCHFAPLTWVCKPQGG;
   GGTYSCHFGPATWVCKPQGG;
   GGTYSCHFGPLTAVCKPQGG;
   GGTYSCHFGPLTFVCKPQGG;
   TYSCHFGPLTWVCKPQ;
   YSCHFGPLTWVCKP;
   SCHFGPLTWVCK;
   GGTYSCFGPLTWVCKPQGG;
   TYSCHFGPLTWVCKPQGG;
   YSCHFGPLTWVC;
   GGTYSCHFGPLTFVCKPQGG; and
   HFGPLTWV.
7. The peptide according to 1, which is selected from the group consisting of the following:
   GGLYLCRFGPVTWDCGYKGG;
   GGTYSCHFGPLTWVCKPQGG;
   GGDYHCRMGPLTWVCKPLGG;
   VGNYMCHFGPITWVCRPGGG;
   GGVYACRMGPITWVCSPLGG;
   VGNYMAHMGPITWVCRPGG;
   GGPHHVYACRMGPLTWIC;
   GGTYSCHFGPLTWVCKPQ;
   GGLYACHMGPMTWVCQPLRG;
   TIAQYICYMGPETWECRPSPKA;
   YSCHFGPLTWVCK;
   YCHFGPLTWVC; and
   HFGPLTWV.
8. The peptide according to 1, of which the sequence of amino acids is cyclized.
9. The peptide according to 8, which is selected from the group consisting of the following:
   GGTYSCHFGPLTWVCKPQGG ;
   GGTYSCHFGPLTWVCKPQ ;
   GGLYACHMGPMTWVCQPLRG;
   TIAQYICYMGPETWECRPSKA;
   YSCHFGPLTWVCK;
   YCHFCGPLTWVC;
   Ac-GGTYSCHFGPLTWVCKPQGG;
   GGCRIGPITWVCGG;
   LGRKYSCHFGPLTWVCQPAKKD;
   GGTASCHFGPLTWVCKPQGG;
   GGNYYCRFGPITFECHPTGG;
   GGEYLCRMGPMTWVCTPVGG;
   GGLYTCRMGPITWVCLPAGG;
   GGTTSCHFGPLTWVCKPQGG;
   GGTFSCHFGPLTWVCKPQGG;
   GGTYSCHFGALTWVCKPQGG;
   GGTYSCHFGPGPLAWVCKPQGG;
   GGTYSCHFAPLTWVCKPQGG;
   GGTYSCHFGPATWVCKPQGG;
   GGTYSCHFGPLTAVCKPQGG;
   GGTYSCHFGPLTFVCKPQGG;
   TYSCHFGPLTWVCKPQ;
   YSCHFGPLTWVCKP;
   YSCHFGALTWVCK;
   SCHFGPLTWVCK;
   GGTYSEHFGPLTWVKKPQGG;
   GGTYSCFGPLTWVCKPQGG ;
   TYSCHFGPLTWCKPQGG;
   YSCHFGPLTWVC;
   GGTYSCHFGPLTFVCKPQGG; and 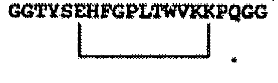
GGTYSEHFGPLTWVKKPQGG
10. The peptide according to 1, of which the sequence of amino acids is dimerized.
11. The peptide according to 10, which comprises the following sequence of amino acids:

GGTYSCHFGPLTWVCKPQGG
     |        |
GGTYSCHFGPLTWVCKPQGG.

In addition to them, an EPO mimic peptide also includes HEMATIDE^{™} developed by Affymax, Inc. and EMP 1 described in Example 2.

An EPO production promoter is a drug which induces the production of biologically-inherent EPO by administering the drug in vivo. For example, it includes a HIF (Hypoxia-Inducible Factor) stabilizer and it may have any structure provided that it has the HIF stabilizing action. More specifically, the example thereof is FG2216 (YM311 by a Japanese name) developed by FibroGen, Inc. Further, it may be an EPO production promoter which has the action mechanism other than that of an inhibitor of hypoxia-inducible factor proline hydroxylase.

Meanwhile, the hematopoietic factor production promoters of the present invention can be used as the active ingredient of pharmaceutical products that are used for treating or preventing various diseases induced by the decrease of erythroid hematopoiesis; or as the constituent of foods or medical foods. The diseases against which the hematopoietic factor production promoters of the present invention are expected to be effective are renal anemia, iron-deficiency anemia, hemolytic anemia, aplastic anemia, pernicious anemia, anemia by the blood loss, and anemia accompanying the therapy with anticancer agents.

As a method of applying the active ingredient of the present invention to a pharmaceutical, oral administration or parenteral administration can be employed. However, upon administration, the active ingredient is mixed with a solid or liquid nontoxic carrier for pharmaceutical use which is suitable for the administration route such as oral administration or injection, whereby it can be administered in a common dosage form for a pharmaceutical preparation. Examples of such a pharmaceutical preparation include solid preparations such as tablets, granules, powders and capsules, liquid preparations such as solutions, suspensions and emulsions, lyophilized preparations and the like. These pharmaceutical preparations can be prepared in a customary manner. Examples of the nontoxic carrier for pharmaceutical use include glucose, lactose, sucrose, starches, mannitol, dextrins, glycerides of fatty acids, polyethylene glycol, hydroxyethyl starches, ethylene glycol, polyoxyethylene sorbitan fatty acid esters, amino acids, gelatin, albumin, water, physiological saline and the like. Further, a commonly used additive such as a stabilizer, a lubricant, an emulsifying agent, a binder or a tonicity adjusting agent can be used as needed.

The present invention provides differentiation inducers of erythroid progenitor cells which have high safety and versatility, and can be orally administered.

The differentiation inducers of erythroid progenitor cells of the present invention are expected to be able to prevent or treat anemia of the patients with anemia and to improve clinical symptoms induced by anemia such as the lowered motivation to work, fatigability, breathlessness, lightheadedness and palpitation. In addition to it, it is expected to be able to surely improve anemia if it can be orally administered, because the oral administration does not accompany pain unlike subcutaneous or intravenous injection, and such drug can be easily taken every day at home without going to hospital. Further, since the differentiation inducers of erythroid progenitor cells of the present invention comprise an amino acid which exists in the body, there is no expression of the side-effects when taking it. Thus, it is expected to be able to treat anemia without concerning about deterioration of renal function.

For example, as for the patients who are under the treatment with rHuEPO, it is expected to reduce the burden of the patients on the cost or pain caused by the subcutaneous or intravenous injection since the required EPO administered dose decreases by additionally administering the active ingredient of the present invention to the patients whose required EPO administered dose is less than the maximal dosage and administration and preferably 1500 to 9000 IU/week. Besides it, it is expected to improve anemia of the EPO unresponsive patients whose required EPO administered dose is the maximal dosage and administration or more and preferably 9000 IU/week or more. Further, in the future, in the case that the treatment with EPO mimic peptides or EPO production promoters becomes widespread, the required EPO administered dose is expected to decrease by additionally administering the active ingredient of the present invention.

By using the hematopoietic factor production promoter of the present invention, an agonist for the promoter, that is, a substance that enhances the hematopoietic factor production-promoting activity that the hematopoietic factor production promoter of the present invention has (hereinafter also referred to as a "hematopoietic factor production-promoting activity enhancer") can be screened. It can be expected that such a hematopoietic factor production-promoting activity enhancer is developed as a therapeutic agent for a disease in the case where the disease is caused by such as lack of promotion of hematopoiesis due to the promotion of hematopoietic factor production by arginine present in vivo. Here, the hematopoietic factor production- promoting activity means an action of elevating production of a certain molecule via the gene expression of a hematopoietic factor.

The hematopoietic factor production-promoting activity enhancer can be screened by, for example, the following steps, however, the method is not limited to these steps:
i) measuring hematopoietic factor production- promoting activities by adding arginine or arginine and a test substance to a hematopoietic factor production- promoting activity measurement system which is for measuring the hematopoietic factor production-promoting activity;
ii) comparing the hematopoietic factor production- promoting activity in the case of adding only arginine to the measurement system with the hematopoietic factor production-promoting activity in the case of adding arginine and a test substance to the measurement system; and
iii) selecting the test substance exhibiting a higher hematopoietic factor production-promoting activity when the test substance is added.

By using the hematopoietic factor production promoter of the present invention, an antagonist for the promoter, that is, a substance that inhibits the hematopoietic factor production-promoting activity that the hematopoietic factor production promoter of the present invention has (hereinafter also referred to as a "hematopoietic factor production-promoting activity inhibitor") can be screened. It can be expected that such a hematopoietic factor production-promoting activity inhibitor is developed as a therapeutic agent for a disease in the case where the promotion of hematopoiesis due to the promotion of hematopoietic factor production by arginine present in vivo is associated with the disease.

The hematopoietic factor production-promoting activity inhibitor can be screened by, for example, the following steps, however, the method is not limited to these steps:
i) measuring hematopoietic factor production- promoting activities by adding arginine or arginine and a test substance to a hematopoietic factor production- promoting activity measurement system which is for measuring the hematopoietic factor production-promoting activity;
ii) comparing the hematopoietic factor production- promoting activity in the case of adding only arginine to the measurement system with the hematopoietic factor production-promoting activity in the case of adding arginine and a test substance to the measurement system; and
iii) selecting the test substance exhibiting a lower hematopoietic factor production-promoting activity when the test substance is added.

The measurement of the hematopoietic factor production-promoting activity is performed by using, for example, cells isolated from an animal. The cells and the hematopoietic factor production-promoting activity measurement system can be used without being particularly limited as long as they enable the detection of gene expression and protein production after the addition of a test substance. As the hematopoietic factor production- promoting activity measurement system, in addition to the quantitative PCR method described in Examples and the like, the gene chip method, the microarray method, in situ hybridization, RNase protection assay, Northern blotting, the differential display method, SDS acrylamide gel electrophoresis, Western blotting, column chromatography and the like can be used.

The hematopoietic factor production promoter according to the present invention may comprise either arginine or a physiologically acceptable salt thereof, or may comprise an arbitrary mixture thereof. Further, the concentration of the test substance may be arbitrary as long as it does not affect the hematopoietic factor production-promoting activity measurement system. Further, the test substance may be a single compound or a mixture or a composition containing a plurality of compounds. Incidentally, arginine and the test substance are generally added to the hematopoietic factor production-promoting activity measurement system simultaneously, however, as long as the hematopoietic factor production-promoting activity of arginine can be detected, they may not be added simultaneously.

By the above-mentioned procedure, or by repeating the above-mentioned procedure, a substance inhibiting or enhancing the hematopoietic factor production-promoting activity that the hematopoietic factor production promoter of the present invention has, a composition containing such a substance or the like can be screened.

The hematopoietic factor production-promoting activity inhibitor and the hematopoietic factor production-promoting activity enhancer selected as described above are expected as a new substance for regulating the hematopoietic factor production-promoting activity of arginine, a therapeutic agent for a variety of diseases associated with the hematopoietic factor production-promoting activity or a candidate thereof.

According to the present invention, a myeloerythroid progenitor cell differentiation inducer which has high safety, can be orally administered and is widely used is provided.

It is expected that the bone marrow erythoid progenitor cell(s) differentiation inducer of the present invention enables prevention or treatment of anemia for patients with anemia, and clinical symptoms such as less incentive to work, fatigability, shortness of breath, lightheadedness and palpitation, each of which accompanies anemia, are improved. Further, if it can be orally administered, the pain due to subcutaneous or intravenous injection is not caused, and moreover, it can be easily taken daily at home without going to the hospital. Therefore, it is expected that the inducer can achieve reliable improvement of anemia. Further, arginine is an amino acid present in vivo, therefore it is expected that side effects accompanying its intake do not occur and treatment can be performed without concerning the worsening of the renal function.

Hereinafter, the present invention will be specifically described with reference to Examples, however, the present invention is not limited to the following Examples.

### Examples

### [Example 1] CFU-E- colony stimulating activity of arginine

An in vitro mouse CFU-E colony assay was carried out in accordance with the following method. After a female BDF-1 mouse at 10 weeks of age (Charles River Japan, Inc.) was killed by the cervical dislocation method, bone marrow cells were isolated from the femur and suspended in an IMDM medium (Invitrogen) containing 10% FCS (JRH Bioscience Inc). The bone marrow cells were centrifuged at 1500 rpm for 10 minutes at 4°C and the precipitated bone marrow cells were resuspended in an amino acid-free IMDM medium and the number of cells was measured. To a dish with a diameter of 3.5 cm (Nalge Nunc, Inc. International), 1 ml of a methyl cellulose semi-solid medium in which the bone marrow cells were suspended {1 IU/ml rHuEPO (Chugai Pharmaceutical Co., Ltd.), 100 µM 2-mercaptoethanol (Wako Pure Chemical Industries, Ltd.), 24% FCS (JRH Bioscience Inc.), 0.8% methyl cellulose (methyl cellulose containing IMDM solution M3134, Stem Cell Technologies, Inc.), 2% BSA (SIGMA-ALDRICH Japan K.K.), 2.2 x 10⁵ cells/ml of bone marrow cells} containing arginine at a concentration of 170 µM was added (which is equal to an IMDM medium whose concentration of the amino acid composition is one-third and which includes FCS at 24%). In addition to the above composition, a medium was prepared by additionally adding arginine so as to give a final concentration of 600 µM, and the culture was carried out under the condition of 37°C and 5% CO₂ for 48 hours. Then, the number of CFU-E colonies was measured using an inverted microscope. N = 4 for each condition. Unpaired t-test was used for a statistical analysis.

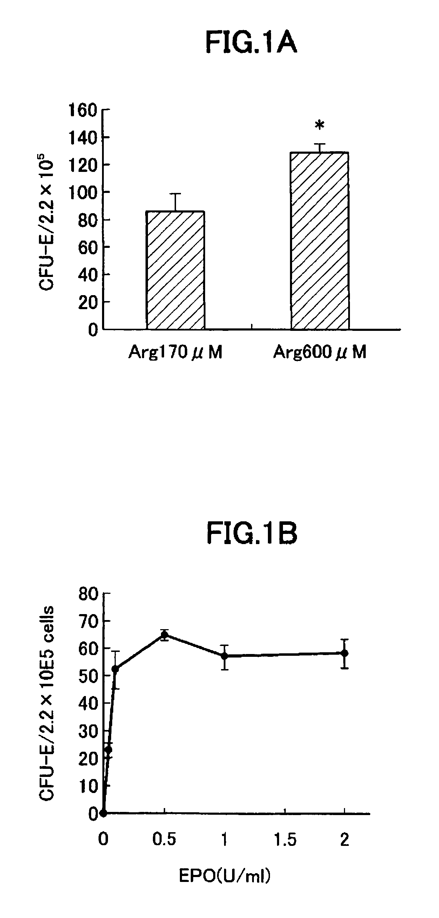

The results of CFU-E colony assay are shown in Fig. 1. In the graph, * indicates P < 0.05. The data is expressed as a mean value ± SEM. The vertical axis indicates the number of CFU-E colonies per 2.2 x 10⁵ bone marrow cells.

In Fig. 1, it is shown that the number of CFU-E increased when arginine was added to give a final concentration of 600 µM. From this, it was revealed that arginine directly acts on a bone marrow cell and increases the number of CFU-E.

From the result that arginine increased the number of CFU-E under the condition in which EPO sufficiently existed, it is expected that arginine has the enhancing action of the effect of EPO and the decreasing effect of EPO administered dose. It is obvious that 1U/mL of EPO is sufficient from the result of Figure 1(B) which was conducted under the same condition as that of Figure 1(A) that 0.5U/mL or more of EPO was the maximal action amount. The combination with an EPO mimic peptide or EPO production promoter reaches the same result, as mentioned below in detail.

It is thought that EPO acts on erythroid hematopoiesis by bonding to an EPO receptor which exists on the erythroid stem cell membrane. As Figure 1(B) shows, EPO increases the erythroid stem cells CFU-E depending on the concentration, and it becomes the maximal activity when each reaches the specified concentration. After that, the maximal activity is kept though the concentration increases. Arginine mentioned in the present invention further increases CFU-E when acting it simultaneously even though such sufficient amount of EPO exists. Thus, it is clarified that each EPO and arginine has a different site of action and both have the synergetic effect.

As mentioned above, EPO unresponsiveness indicates the state wherein the enough effect does not express though EPO is administered in the amount thought to be effective. Even in such a case that the enough effect cannot be obtained by the administration of EPO only, the higher effect beyond the EPO maximal activity can be obtained by administering arginine. Further, in the patients not having EPO unresponsiveness, when their required EPO administered dose is much, the effect of EPO can be enhanced by combining arginine and, as a result, the EPO administered dose can be decreased. Namely, it is usable as an "enhancer of the effect of EPO" or an "agent which decreases the EPO administered dose".

Since the site of action of EPO and an EPO mimic peptide is the same EPO receptor, the relationship between EPO and arginine mentioned above also applies to an EPO mimic peptide and arginine. Namely, it is usable as an "enhancer of the effect of an EPO mimic peptide" or an "agent which decreases the administered dose of an EPO mimic peptide".

In the body, EPO is produced in the kidney, and in the case that EPO required for the erythroid hematopoiesis is deficient due to the diseases such as renal anemia, EPO as a pharmaceutical product is administered. However, even though one has renal anemia, it is not to say that their kidney cannot produce EPO. If EPO production can be increased by using a renal EPO production promoter, the same therapeutic effect can be expected as that of the EPO administration. Arginine in the present invention can act equally on EPO administered as a pharmaceutical product and inherent EPO of which production is promoted from the kidney. Therefore, it is possible to combine arginine with an EPO production promoter(s) such as FG2216. In such a case, the effect shown in Figure 1 is expected, and it is possible to enhance the effect than the sole administration and to decrease the administered dose. Namely, it is usable as an "enhancer of the effect of an EPO production promoter" or an "agent which decreases the administered dose of an EPO production promoter".

### [Example 2] The CFU-E increasing action of arginine (EPO mimic peptide)

As a typical example of an EPO mimic peptide, EMP 1 (GGTYSCHFGPLTWVCKPQGG-NH₂, C6-C15 disulfide bonding) described in the cited reference (Table 1 of Science Vol. 273, 458-463, 1996, etc) was prepared by using the method described in the cited reference (3700-3701 pages of Biochemistry Vol. 37(11), 3699-3710).

After making a female BDF-1 mouse of 10 weeks old (by Charles River Laboratories Japan, Inc.) die by the cervical dislocation method, the bone-marrow cells were isolated from the thighbone and suspended in the IMDM medium (by Invitrogen Corporation) which contained 10% FCS (by JRH Biosciences). The bone-marrow cells were centrifuged at 1500rpm for 10 minutes at 4°C. Then, the precipitated bone-marrow cells were substituted on the IMDM medium without an amino acid, and the number of the cells was determined. 1mL of a methylcellulose semisolid medium containing 170 µ M of arginine concentration wherein the bone-marrow cells were suspended (30 µ M of EMP1; 100 µ M 2-mercaptoethanol by Wako Pure Chemical Industries, Ltd.; 24% FCS by JRH Biosciences; 0.8% methylcellulose, IMDM solution M3134 containing methylcellulose by StemCell Technologies Inc.; 2% BSA by Sigma-Aldrich Japan K.K.; and bone-marrow cells 2.2×10⁵/mL) was added to a dish of 3.5cm in diameter (Nalge Nunc International K.K.) (equivalent to the IMDM medium wherein the concentration of the amino acid composition containing 24% FCS is 1/3). In addition to the above composition, the medium to which arginine was further added so that its final concentration became 770 µ M was prepared and cultured at 37°C under 5% CO₂ for 48 hours. Then, the number of CFU-E colonies was determined by using an inverted microscope. N = 4 in each condition. The unpaired t-test was used as the statistical test.

From the result of the CFU-E colony assay, it was clarified that arginine had an increasing effect of CFU-E under the existence of EMP1 in its maximal action concentration.

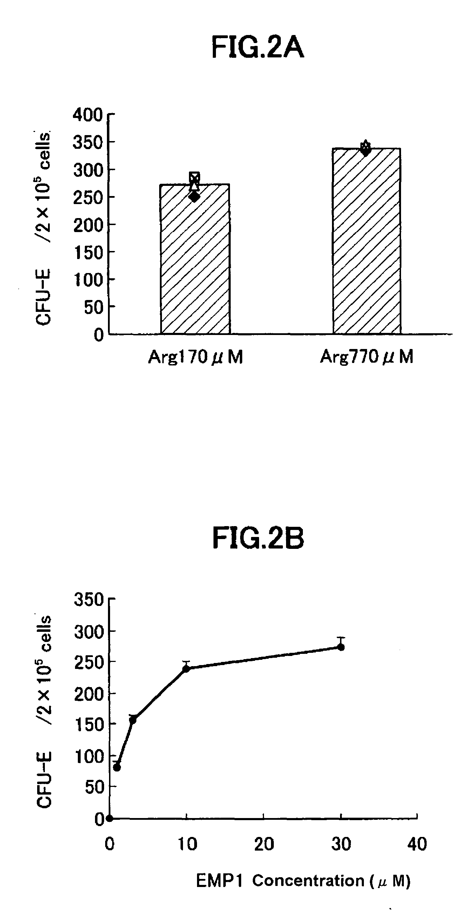

Since arginine increased the number of CFU-E under the condition in which an EPO mimic peptide sufficiently existed, it is expected that arginine has the enhancing action of the effect of an EPO mimic peptide and the decreasing effect of the administered dose of an EPO mimic peptide. It is obvious that 30 µ M of the EPO mimic peptide is sufficient from the result of Figure 2(B) which was conducted under the same condition as that of Figure 2(A), that 10 µM or more of an EPO mimic peptide was the maximal action amount.

### [Example 3] Activin, SCF and IGF-1 production-promoting activity of arginine in bone marrow cells isolated from renal failure rat

The production of a renal failure rat (a rat from which 5/6 of kidney was removed) was carried out as follows. A male Wistar rat at 8 weeks of age (Charles River Japan, Inc.) was subjected to the total extirpation of the right kidney and the 2/3 extirpation of the renal cortex of the left kidney under anesthesia with Nembutal and was sutured. After the surgery, the rat was housed under normal housing condition (CRF-1 diet, Charles River Japan, Inc.) and 6 weeks after the surgery, the renal failure rat was killed by diethyl ether, then the both femurs were excised and bone marrow cells were isolated. The isolated bone marrow cells were suspended in an IMDM medium with blood amino acid concentration containing 10% FCS. As for the IMDM medium with blood amino acid concentration, an IMDM medium in which the amino acid composition was changed to a composition shown in Table 1 was prepared and used.

Ten milliliters of the bone marrow cell suspension prepared at 2 x 10⁶ cells/ml was cultured in a dish with a diameter of 10 cm (Nalg Nunc, Inc.) under the condition of 37°C and 5% CO₂ for 24 hours. Twenty-four hours after the initiation of culture, arginine was added thereto. After 2 hours, the bone marrow cells in the dish were colleted and used for preparation of total RNA and cDNA synthesis.

**Table 1**

| No. | Amino acid | Concentration(µM) |
|---|---|---|
| 1 | L-Arginine | 100 |
| 2 | L-Asparagine | 50 |
| 3 | L-Aspartic acid | 10 |
| 4 | L-Cystine | 25 |
| 5 | L-Glutamic acid | 60 |
| 6 | L-Glutamine | 600 |
| 7 | Glycine | 250 |
| 8 | L-Histidine | 70 |
| 9 | Alanine | 387 |
| 10 | L-Isoleucine | 75 |
| 11 | L-Leucine | 130 |
| 12 | L-Lysine | 260 |
| 13 | L-Methionine | 45 |
| 14 | L-Phenylalanine | 60 |
| 15 | L-Proline | 130 |
| 16 | L-Serine | 140 |
| 17 | L-Threonine | 160 |
| 18 | L-Tryptophan | 50 |
| 19 | L-Tyrosine | 70 |
| 20 | L-Valine | 200 |

Total RNA preparation and cDNA synthesis were carried out by the following methods. Bone marrow cells corresponding to one femur were suspended in 1.5 ml of Isogen (Nippon Gene Co., Ltd.) and homogenized (BIO 101 FastPrep). Total RNA was purified using 1 ml of the homogenate in accordance with the method described in Isogen. The quality of the total RNA was confirmed by Bioanalyzer (Japan Becton Dickinson). By using 5 µg of the total RNA as a template, cDNA synthesis was carried out using an oligo dT 20mer primer (Invitrogen) and SuperScript III (Invitrogen). The method of cDNA synthesis was in accordance with the method described in SuperScript III.

The quantification of mRNA of each of the hematopoietic factors was carried out by the quantitative PCR method mentioned below. Based on the sequences of activin (rat Inhibin betaA), SCF and IGF-1 registered in NCBI (GenBank accession Nos. M37482, NM021843, and NM178886, respectively in the order), oligonucleotide primers to be used for the quantitative PCR method were synthesized. The primers described in SEQ ID NOS: 1 and 2 in Table 2, the primers described in SEQ ID NOS: 3 and 4 in Table 2, and the primers described in SEQ ID NOS: 5 and 6 in Table 2 were used for activin gene, SCF gene, and IGF-1 gene, respectively. The quantitative PCR method was in accordance with the method described in SYBRGreen PCR kit (TOYOBO., Ltd.). More specifically, a PCR reaction (after 1 minute at 94°C, 40 cycles of 30 seconds at 94°C and 1 minute at 60°C) with an ABI7700 (Applied Biosystems Japan Ltd.) using 1 µl of a cDNA solution as a template, 500 nM of the primers, and a SYBRGreen PCR kit and the detection of amplified product were carried out and the relative concentration of mRNA was calculated. After the quantitative PCR, the presence or absence and size of an amplified product were confirmed by agarose gel electrophoresis.

**Table 2**

| SED ID No. | Oligonucleotide primer |
|---|---|
| 1 | gaaaacgggtatgtggaga |
| 2 | tgaaacagacggatggtga |
| 3 | tggtggacgctcttcagtt |
| 4 | catctccagcctcctcaga |
| 5 | ctacaatggacagcaat |
| 6 | gaaactctctctctttctgttgc |

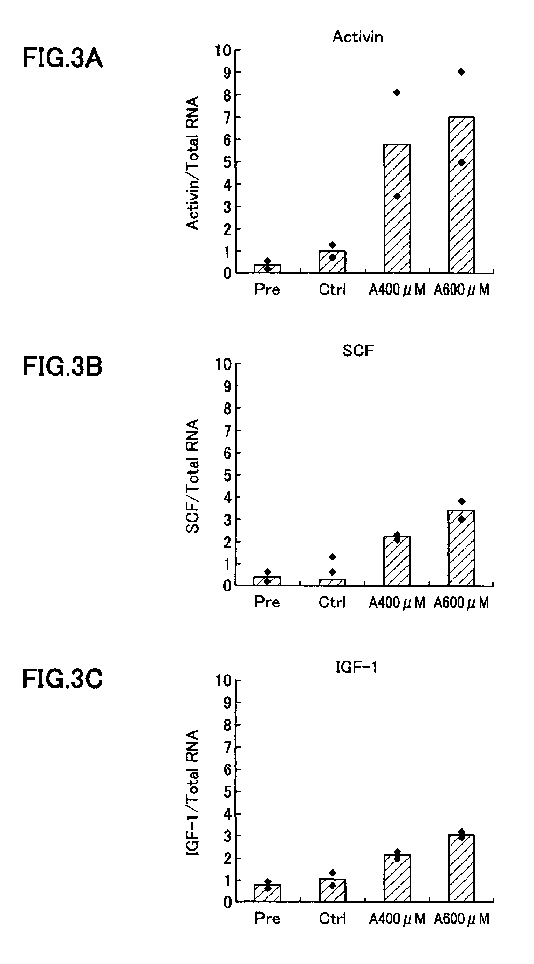

Bone marrow cells were isolated from a renal failure rat, and the bone marrow cells were collected 2 hours after the addition of arginine, and then mRNA of each of activin, SCF and IGF-1 was quantified by the quantitative PCR method. The results are shown in Fig 3. The data is expressed as a mean value ± SEM. The vertical axis indicates a relative mRNA level per total RNA.
Pre: Before the addition of arginine, arginine concentration: 100 µM
Ctrl: 2 hours without the addition of arginine, arginine concentration: 100 µM
A400 µM: 2 hours with the addition of arginine, arginine concentration: 400 µM
A600 µM: 2 hours with the addition of arginine, arginine concentration: 600 µM

From these results, it was revealed that arginine directly acts on a bone marrow cell and promotes the production of activin, SCF and IGF-1.

### [Example 4] Inhibition of CFU-E-colony stimulating activity of arginine by activin, SCF, or IGF-1 inhibitor

An in vitro mouse CFU-E colony assay with a female BDF-1 mouse at 10 weeks of age (Charles River Japan, Inc.) was carried out in accordance with the method described in Example 1. Under the presence of 170 µM arginine, arginine was additionally added to make a final concentration of 600 µM and follistatin (FSN, anti-activin, R&D Co., 100 ng/ml), anti-SCF antibody (Pepro Tech EC Ltd., 1 µg/ml) and anti-IGF-1 antibody (Pepro Tech EC Ltd., 2 µg/ml) was further added. At 28 hours after the culture was carried out under the condition of 37°C and 5% CO₂, the number of CFU-E colonies was measured using an inverted microscope. N = 4 for each condition. Unpaired t-test was used for a statistical analysis. * indicates P < 0.05, and ** indicates P < 0.005. The data is expressed as a mean value ± SEM.

The results of CFU-E colony assay are shown in Fig. 3. The vertical axis indicates the number of CFU-E colonies per 2.2 x 10⁵ bone marrow cells.

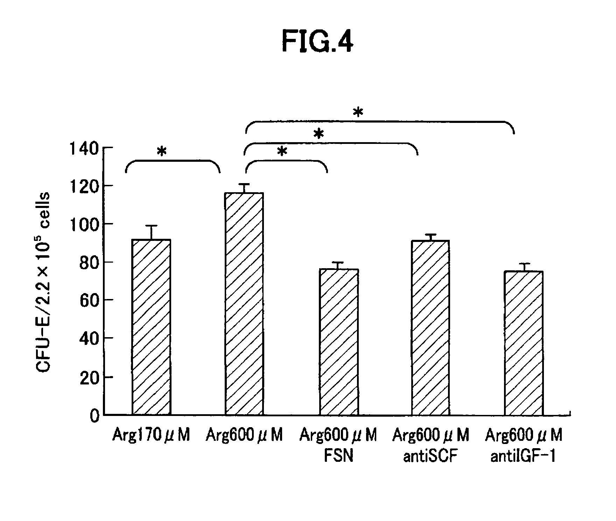

From Fig. 4, it is found that the CFU-E-colony stimulating activity of arginine is inhibited by follistatin, anti-SCF antibody and anti-IGF-1 antibody. Accordingly, it was revealed that arginine acts on a bone marrow cell and increases CFU-E via the promotion of production of activin, SCF and IGF-1, which are hematopoietic factors.

Sequence Listing

<110> Ajinomoto Co. Inc.
<120> Bone marrow erythroid progenitor cell(s) differentiation
inducer
<130> Y1N-0092
<160> 43
<210> 1
<211> 19
<212> nucleic acid
<400> 1
gaaaacgggtatgtggaga

<210> 2
<211> 19
<212> nucleic acid
<400> 2
tgaaacagacggatggtga

<210> 3
<211> 19
<212> nucleic acid
<400> 3
tggtggacgctcttcagtt

<210> 4
<211> 19
<212> nucleic acid
<400> 4

catctccagcctcctcaga

<210> 5
<211> 17
<212> nucleic acid
<400> 5
ctacaatggacagcaat

<210> 6
<211> 23
<212> nucleic acid
<400> 6
gaaactctctctctttctgttgc

<210> 7
<211> 20
<212> PRT
<400> 7
gglylcrfgpvtwdcgykgg

<210> 8
<211> 20
<212> PRT
<400> 8
ggtyschfgpltwvckpqgg

<210> 9
<211> 18
<212> PRT

<400> 9
ggtyschfgpltwvckpq

<210> 10
<211> 20
<212> PRT
<400> 10
ggdyhcrmgpltwvckplgg

<210> 11
<211> 20
<212> PRT
<400> 11
vgnymchfgpitwvcrpggg

<210> 12
<211> 20
<212> PRT
<400> 12
ggnymchfgpitwvcrpggg

<210> 13
<211> 20
<212> PRT
<400> 13
ggvyacrmgpitwvcsplgg

<210> 14
<211> 19

<212> PRT
<400> 14
vgnymahmgpitwvcrpgg

<210> 15
<211> 18
<212> PRT
<400> 15
ggphhvyacrmgpltwic

<210> 16
<211> 18
<212> PRT
<400> 16
ggtyschfgpltwvckpq

<210> 17
<211> 20
<212> PRT
<400> 17
gglyachmgpmtwvcqplrg

<210> 18
<211> 22
<212> PRT
<400> 18
tiaqyicymgpetwecrpspka

<210> 19

<211> 13
<212> PRT
<400> 19
yschfgpltwvck

<210> 20
<211> 11
<212> PRT
<400> 20
ychfgpltwvc

<210> 21
<211> 20
<212> PRT
<400> 21
ggtyschfgpltwvckpqgg

<210> 22
<211> 14
<212> PRT
<400> 22
ggcrigpitwvcgg

<210> 23
<211> 22
<212> PRT
<400> 23
lgrkyschfgpltwvcqpakkd

<210> 24
<211> 20
<212> PRT
<400> 24
ggtaschfgpltwvckpqgg

<210> 25
<211> 20
<212> PRT
<400> 25
ggnyycrfgpitfechptgg

<210> 26
<211> 20
<212> PRT
<400> 26
ggeylcrmgpmtwvctpvgg

<210> 27
<211> 20
<212> PRT
<400> 27
gglytcrmgpitwvclpagg

<210> 28
<211> 20
<212> PRT
<400> 28
ggttschfgpltwvckpqgg

<210> 29
<211> 20
<212> PRT
<400> 29
ggtfschfgpltwvckpqgg

<210> 30
<211> 20
<212> PRT
<400> 30
ggtyschfgaltwvckpqgg

<210> 31
<211> 20
<212> PRT
<400> 31
ggtyschfgplawvckpqgg

<210> 32
<211> 20
<212> PRT
<400> 32
ggtyschfapltwvckpqgg

<210> 33
<211> 20
<212> PRT
<400> 33

ggtyschfgpatwvckpqgg

<210> 34
<211> 20
<212> PRT
<400> 34
ggtyschfgpltavckpqgg

<210> 35
<211> 20
<212> PRT
<400> 35
ggtyschfgpltfvckpqgg

<210> 36
<211> 16
<212> PRT
<400> 36
tyschfgpltwvckpq

<210> 37
<211> 14
<212> PRT
<400> 37
yschfgpltwvckp

<210> 38
<211> 12
<212> PRT

<400> 38
schfgpltwvck

<210> 39
<211> 19
<212> PRT
<400> 39
ggtyscfgpltwvckpqgg

<210> 40
<211> 18
<212> PRT
<400> 40
tyschfgpltwvckpqgg

<210> 41
<211> 12
<212> PRT
<400> 41
yschfgpltwvc

<210> 42
<211> 20
<212> PRT
<400> 42
ggtyschfgpltfvckpqgg

<210> 43
<211> 8

<212> PRT
<400> 43
hfgpltwv

## Claims

1. A bone marrow erythoid progenitor cell(s) differentiation inducer, comprising arginine as an active ingredient.

2. A therapeutic agent for anemia, comprising the differentiation inducer according to claim 1.

3. The therapeutic agent for anemia according to claim 2, which is for hemodialysis or peritoneal dialysis.

4. The therapeutic agent for anemia according to claim 2, which is for oral administration.

5. The therapeutic agent for anemia according to claim 2, which is for enteral administration.

6. The therapeutic agent for anemia according to any one of claims 2 to 5, which is for treating renal anemia.

7. The therapeutic agent for anemia according to any one of claims 2 to 5, which is for erythropoietin- unresponsive patients.

8. The therapeutic agent for anemia according to any one of claims 2 to 7, further comprising erythropoietin in combination.

9. The therapeutic agent for anemia according to claim 8, wherein the erythropoietin-like substance(s) is erythropoietin.

10. The therapeutic agent for anemia according to claim 8, wherein the erythropoietin-like substance(s) is an erythropoietin mimic peptide.

11. The therapeutic agent for anemia according to claim 8, wherein the erythropoietin-like substance(s) is an erythropoietin production promoter.

12. The therapeutic agent for anemia according to any one of claims 8 to 11, which is a combination preparation.

13. The therapeutic agent for anemia according to claim 8, which is a kit composed of a medicament comprising arginine and a medicament comprising erythropoietin-like substance(s).

14. The therapeutic agent for anemia according to claim 13, wherein the erythropoietin-like substance(s) is erythropoietin.

15. The therapeutic agent for anemia according to claim 13, wherein the erythropoietin-like substance(s) is an erythropoietin mimic peptide.

16. The therapeutic agent for anemia according to claim 13, wherein the erythropoietin-like substance(s) is an erythropoietin production promoter.

17. An agent for enhancing the effect of erythropoietin-like substance(s), comprising arginine as an active ingredient.

18. The agent according to claim 17, wherein the erythropoietin-like substance(s) is erythropoietin.

19. The agent according to claim 17, wherein the erythropoietin-like substance(s) is an erythropoietin mimic peptide.

20. The agent according to claim 17, wherein the erythropoietin-like substance(s) is an erythropoietin production promoter.

21. An agent for reducing the dose of erythropoietin-like substance(s), comprising arginine as an active ingredient.

22. The agent according to claim 21, wherein the erythropoietin-like substance(s) is erythropoietin.

23. The agent according to claim 21, wherein the erythropoietin-like substance(s) is an erythropoietin mimic peptide.

24. The agent according to claim 21, wherein the erythropoietin-like substance(s) is an erythropoietin production promoter.

25. A hematopoiesis promoter for an individual who stores his/her own blood, comprising arginine as an active ingredient.

26. A hematopoietic factor production promoter comprising arginine as an active ingredient.

27. The hematopoietic factor production promoter according to claim 26, wherein the hematopoietic factor is at least one selected from the group consisting of activin, SCF and IGF-1.

28. The hematopoietic factor production promoter according to claim 26, which promotes production of hematopoietic factor in the bone marrow.

29. A method of screening an enhancer for the hematopoietic factor production promoter according to claim 26, comprising the steps of:
comparing hematopoietic factor production- promoting activities when arginine or arginine and a test substance is/are added to a hematopoietic factor production-promoting activity measurement system which is for measuring a hematopoietic factor production-promoting activity; and
selecting the test substance exhibiting a higher hematopoietic factor production-promoting activity when the test substance is added.

30. A method of screening an inhibitor for the hematopoietic factor production promoter according to claim 26, comprising the steps of:
comparing hematopoietic factor production-promoting activities when arginine or arginine and a test substance is/are added to a hematopoietic factor production-promoting activity measurement system which is for measuring a hematopoietic factor production-promoting activity; and
selecting the test substance exhibiting a lower hematopoietic factor production-promoting activity when the test substance is added.
